# FASCICULE DE BREVET EUROPEEN

(11) **EP 0 699 426 B1**
(45) Date de publication et mention de la délivrance du brevet: **03.05.2000**
(21) Numéro de dépôt: 95420245.3
(22) Date de dépôt: 29.08.1995
(51) Int. Cl.: A61F 2/44

(54) **Prothèse rachidienne intervertébrale à glissement, notamment cervicale**
Gleitzwischenwirbelprothese, insbesondere Halszwischenwirbel
Sliding intervertebral prosthesis, especially cervical intervertebral prosthesis

(30) Priorité: 05.09.1994 FR 9410872
(43) Date de publication de la demande: 06.03.1996
(73) Titulaire: MEDINOV S.A., F-42312 Roanne (FR)
(72) Inventeur: Feron, Jean Marc, F-94300 Vincennes (FR); Kehr, Pierre, F-67000 Strasbourg (FR); Nazarian, Serge, F-13008 Marseille (FR); Graftiaux, Alain, F-67000 Strasbourg (FR); Ricart, Olivier, Dudelange, 3514 (LU)
(74) Mandataire: Moncheny, Michel

(56) Documents cités:
- EP-A- 0 329 854
- WO-A-91/13598
- WO-A-93/10725
- US-A- 4 759 769
- US-A- 5 258 031

## Description

L'invention se rattache, de manière générale, au secteur technique des prothèses intervertébrales.

Actuellement, pour traiter la dégénérescence discale, on utilise des moyens chimiques et, le plus souvent, des moyens prothétiques. Les moyens prothétiques permettent le montage d'un greffon osseux, entre les plateaux vertébraux. Cette solution donne des résultats satisfaisants mais nécessite le blocage des deux vertèbres considérées, dans le cas notamment des vertèbres cervicales.

On peut citer par exemple, l'enseignement du brevet FR 2570594 qui concerne une prothèse vertébrable mettant en oeuvre une plaquette fixée sur deux vertèbres distincts, la zone médiane de la plaquette portant transversalement, par avance, un greffon.

Le problème que se propose de résoudre l'invention, est de pouvoir redonner une certaine mobilité des deux vertèbres qui ont subi cette dégénérescence discale. Un tel problème se pose plus particulièrement au niveau des vertèbres cervicales, lesquelles sont soumises à des mouvements d'articulation beaucoup plus prononcés que dans le cas des vertèbres lombaires et thoraciques.

En effet, au niveau lombaire et thoracique, on a proposé des éléments prothétiques qui permettent une articulation entre les deux vertèbres considérées. Généralement, ce type d'éléments prothétiques, est composé de deux plateaux métalliques fixés sur les corps vertébraux, entre ces deux plateaux étant monté un implant en silicone.

Cette solution n'est cependant pas transposable pour les vertèbres cervicales, étant donné que le silicone utilisé, malgré de nombreuses recherches effectuées, n'est pas biocompatible dans le temps. Dans ces conditions, le silicone peut se mettre sous forme particulaire, en étant ainsi susceptible de se répartir n'importe où, notamment au niveau du canal de la moelle épinière.

En outre, ce type d'implant nécessite un encombrement relativement important, limitant, de manière importante, les débattements angulaires. Or, si au niveau lombaire et thoracique, la mobilité entre deux vertèbres est relativement peu importante, ce n'est pas le cas au niveau cervical.

Cet état de la technique peut être illustré par l'enseignement du brevet US-A-5258031, qui décrit un ensemble prothétique rachidien comprenant deux implants complémentaires disposés en regard l'un de l'autre, avec capacité d'articulation. La conception de chaque implant permet une articulation dans un seul plan et non pas selon une pluralité de directions, ce qui est le cas au niveau cervical. Cet ensemble prothétique est limité au niveau lombaire. On note également qu'avec ce type de prothèse, l'un des implants, compte-tenu de sa conception, ne peut pas coopérer directement avec la surface cartilagineuse de la vertèbre disposée en regard.

Le problème que se propose donc de résoudre l'invention, est de créer une prothèse rachidienne apte à conserver la mobilité entre deux vertèbres cervicales sans modifier les surfaces cartilagineuses de l'une ou l'autre des vertèbres prothésées, en ayant pour objectif d'obtenir une prothèse modulaire.

Pour résoudre un tel problème, il a été conçu et mis au point une prothèse cervicale à glissement qui comprend une partie constituée d'un élément support présentant une plaque cintrée pour correspondre très sensiblement au profil externe de l'un des corps vertébraux, ladite plaque délimitant deux pattes de fixation, ledit élément présente, dans un plan très sensiblement horizontal, une partie sous forme d'un implant profilé, apte à coopérer aussi bien avec la surface articulaire fibro-cartilagineuse du corps vertébral disposé en regard, qu'avec une partie complémentaire que présente une autre partie rendue solidaire dudit corps vertébral.

Pour résoudre le problème posé de permettre le montage de l'implant profilé, l'agencement de l'élément support est constitué par un anneau ouvert prolongé, à l'opposé de son ouverture et dans un plan sensiblement orthogonal, par la plaque cintrée.

Avantageusement, l'implant a une forme générale, vue en plan, très sensiblement circulaire. La face de dessus de l'implant a un profil en relief très sensiblement complémentaire au profil en creux du corps vertébral avec lequel il coopère.

A noter que dans le cas d'une intervention de reprise, dans l'hypothèse où la surface articulaire fibrocartilagineuse du corps vertébral disposé en regard présente une usure ou des douleurs diverses, le corps vertébral correspondant peut, dans ce cas, être équipé d'un ensemble prothétique complémentaire. A cet effet, l'ensemble comprend, comme précédemment, une partie apte à être rendue solidaire du corps vertébral correspondant et une partie constituant une surface d'appui et d'articulation apte à coopérer avec la surface d'appui de la partie disposée en regard que présente l'autre corps vertébral.

L'invention est exposée, ci-après plus en détail à l'aide des dessins annexés, dans lesquels:

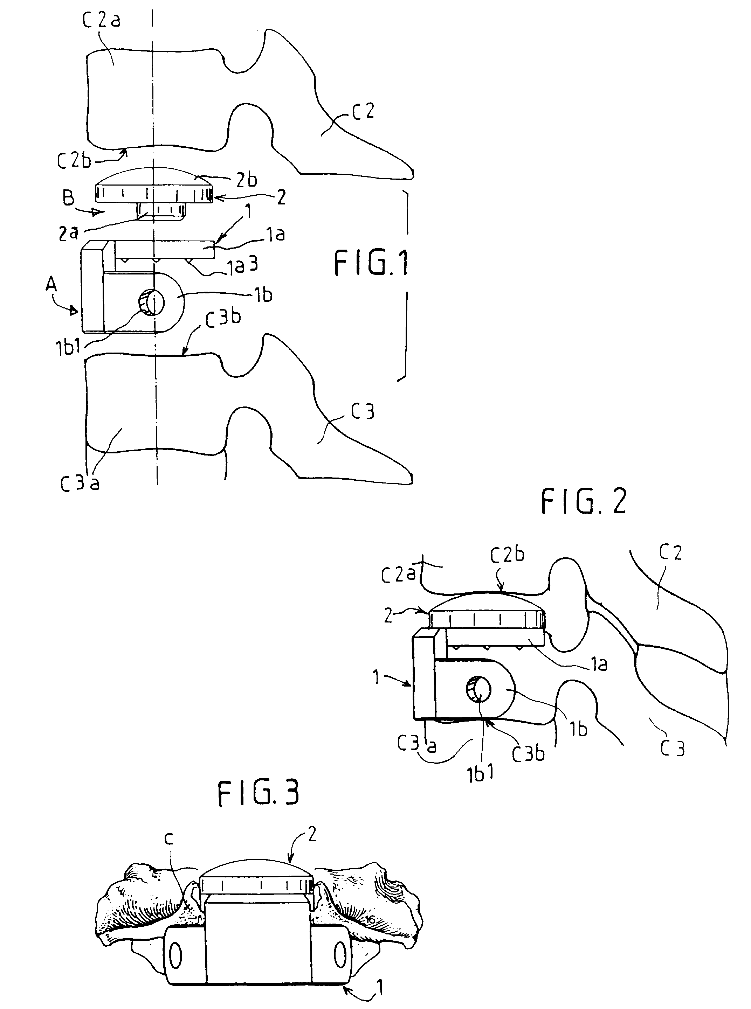

La figure 1 est une vue montrant, avant montage, les éléments constitutifs de la prothèse, et avant fixation entre deux vertèbres cervicales.

La figure 2 est une vue correspondant à la figure 1, après montage et fixation de la prothèse.

La figure 3 est une vue de côté correspondant à la figure 2.

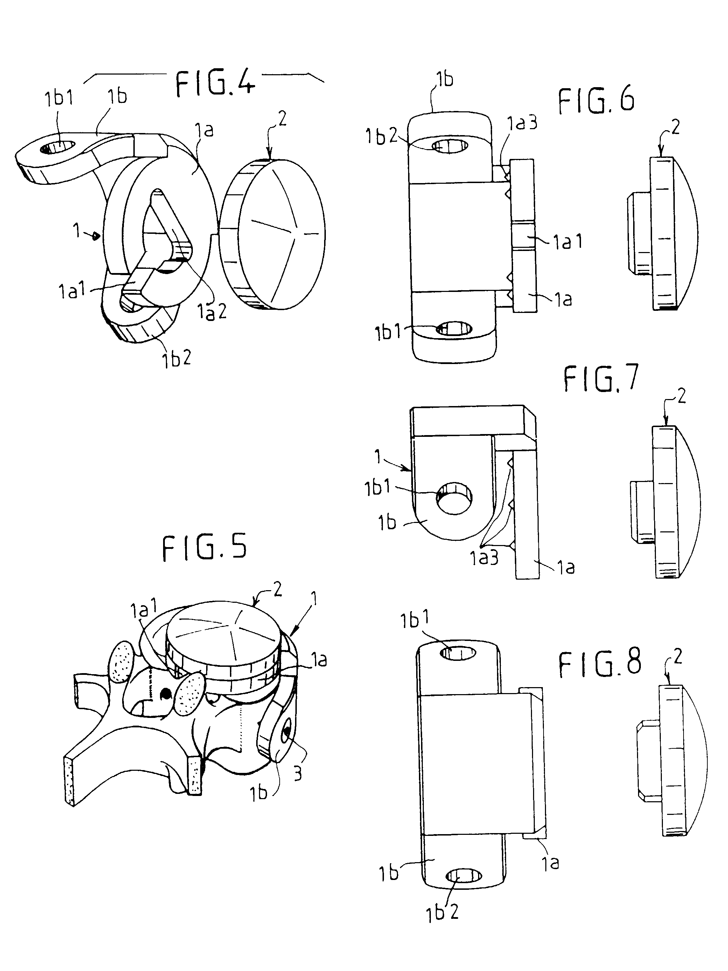

La figure 4 est une vue en perspective avant montage, des éléments constitutifs de la prothèse.

La figure 5 est une vue en perspective d'une vertèbre cervicale équipée de la prothèse selon l'invention.

La figure 6 est une vue de face de la prothèse avant montage des éléments constitutifs.

Les figures 7 et 8 sont respectivement des vues de côté et arrière correspondant à la figure 6.

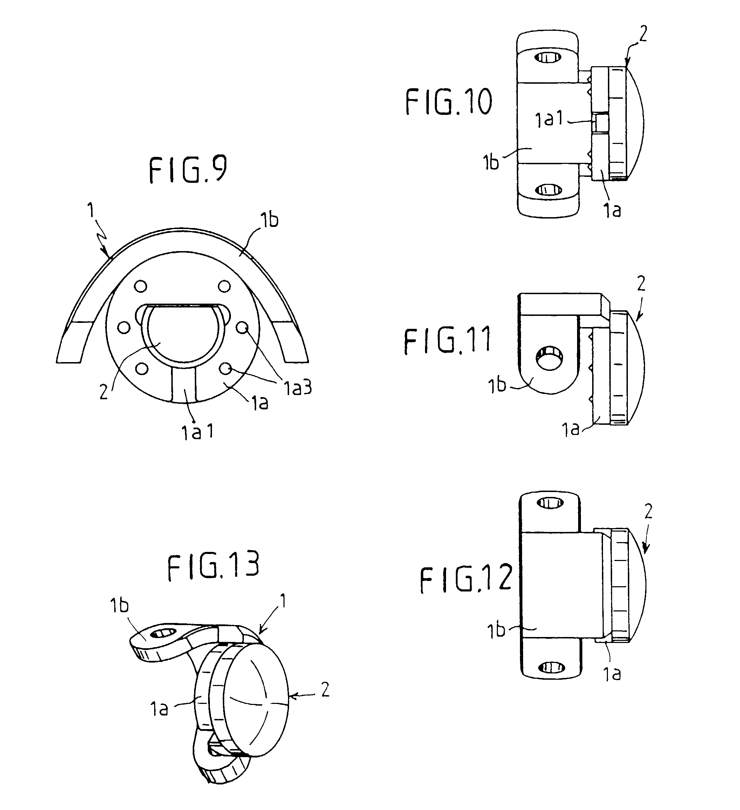

La figure 9 est une vue de dessus de l'élément support.

Les figures 10, 11 et 12 sont des vues correspondant respectivement aux figures 6, 7 et 8 après montage de l'implant sur l'élément support.

La figure 13 est une vue semblable à la figure 4, après montage de l'implant sur l'élément support.

La figure 14 est une vue montrant avant montage, les éléments constitutifs de la prothèse, avant fixation entre deux vertèbres cervicales et dans une autre forme de réalisation.

Les figures 15, 16 et 17 montrent, par des vues de face et de côté, l'implant de reprise.

La figure 18 est une vue montrant le montage de la prothèse, directement en combinaison avec la surface articulaire fibrocartilagineuse du corps vertébral.

La figure 19 est une vue semblable à la figure 18, dans le cas d'une reprise.

Comme indiqué, la prothèse rachidienne selon l'invention, est conformée pour être montée entre deux vertèbres cervicales quelconques, (C2) (C3) par exemple. La prothèse comprend deux parties désignées dans leur ensemble par (A) et (B). La partie (A) est conformée pour être rendue solidaire de l'un des corps vertébraux (C2a) ou (C3a) des vertèbres (C2) ou (C3). L'autre partie (B) constitue une surface d'appui et d'articulation, apte à coopérer avec la surface articulaire fibrocartilagineuse (C3b) ou (C2b) du corps vertébral (C3a) ou (C2a) disposé en regard et recevant la partie (A).

Avantageusement, mais non limitativement, la partie (A) est fixée sur le corps vertébral (C3a) de la prothèse inférieure (C3), tandis que la partie (B) coopère avec la surface articulaire fibrocartilagineuse (C2b) du corps vertébral (C2a) de la prothèse supérieure (C2).

Les deux parties (A) et (B) peuvent constituer un ensemble monobloc ou un ensemble modulaire. Les figures des dessins montrent une prothèse où les deux parties (A) et (B) sont distinctes pour constituer un ensemble modulaire.

La partie (A) comprend un élément support (1) conformé pour être fixé sur l'un des corps vertébraux (C3a) par exemple. La partie supérieure de l'élément (1) présente un anneau ouvert (1a) pour le montage, dans un plan très sensiblement horizontal, d'un implant profilé (2) qui, en l'espèce, constitue la partie (B). L'anneau (1a) présente une ouverture radiale (1a1) et une empreinte débouchante (1a2), apte à recevoir, comme il sera indiqué dans la suite de la description, l'implant (2). A l'opposé de son ouverture (1a1), l'anneau (1a) est prolongé dans un plan sensiblement orthogonal par une plaque cintrée (1b), pour correspondre très sensiblement au profil externe du corps vertébral considéré (C3a). Cette plaque (1b) délimite deux pattes de fixation (1b1) (1b2) susceptibles de recevoir des vis (3) destinées à coopérer avec le corps vertébral correspondant (C3a).

L'anneau (1a) prend appui sur la face articulaire du corps vertébral (C3a) recevant l'élément (1). A cet égard, la face de dessous de l'anneau (1a) peut présenter des aspérités d'ancrage (1a3).

L'implant (2) présente un plot de centrage et de fixation (2a) apte à être emmanché à force notamment, dans l'empreinte profilée (1a2) de l'anneau (1a), l'ouverture radiale (1a1) permettant la déformation élastique de l'anneau. Cet implant (2) a une forme générale, vue en plan, très sensiblement circulaire. La face de dessus (2b) de l'implant est convexe et présente un profil en relief très sensiblement complémentaire au profil en creux concave de la surface articulaire fibrocartilagineuse (C2b) du corps vertébral disposé en regard (C2a). Cet implant (2) peut être exécuté dans différents matériaux (céramique, métallique...).

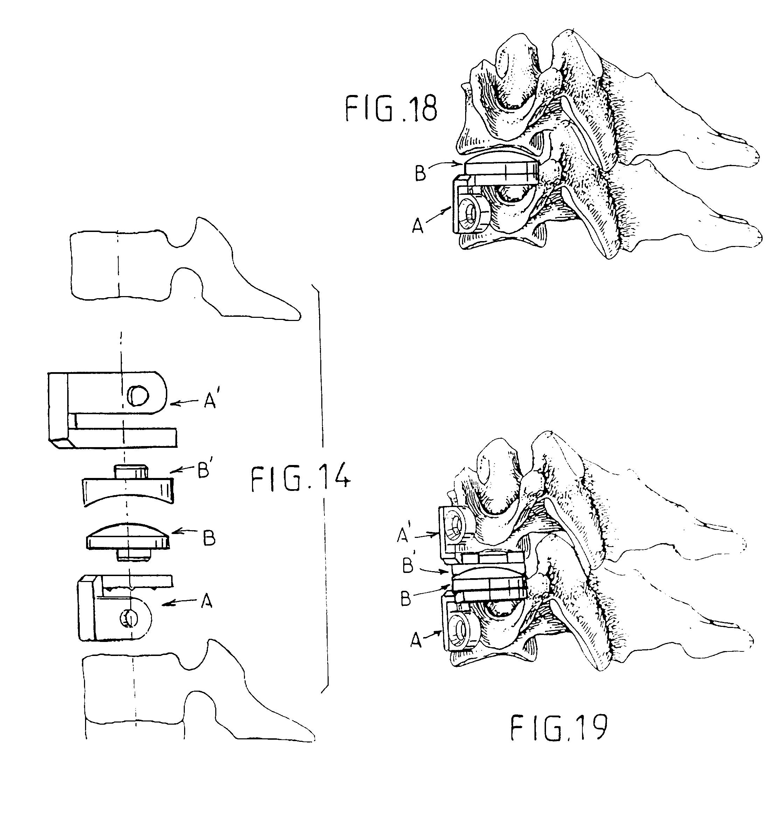

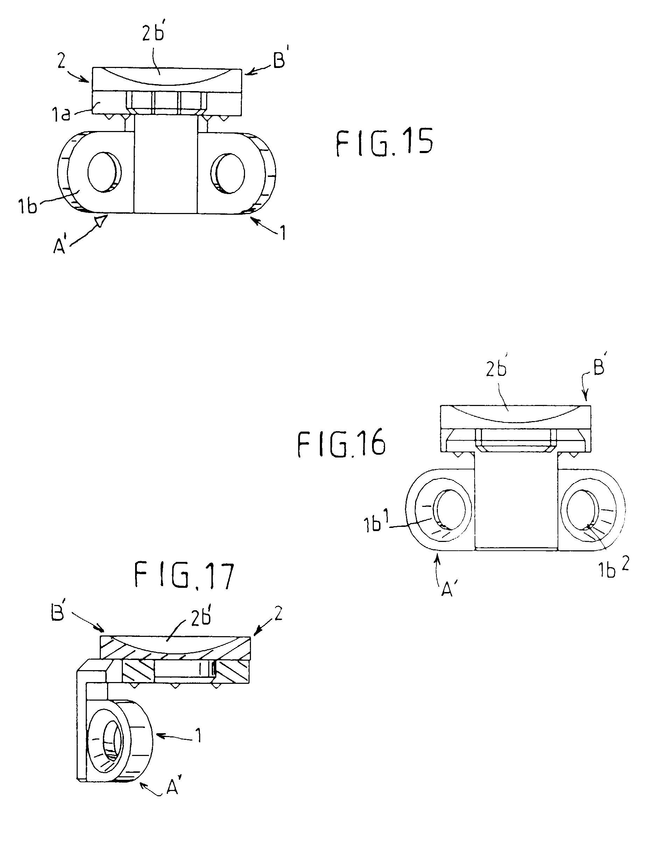

Dans le cas où la surface articulaire fibrocartilagineuse (C2b) du corps vertébral (C2a) n'est pas suffisamment saine, le corps vertébral est équipé d'une prothèse identique à celle décrite, c'est-à-dire avec deux parties désignées dans leur ensemble par (A') et (B'). La partie (A') est strictement identique à la partie (A). Les mêmes repères sont donc utilisés pour désigner les éléments constitutifs de cette partie (A'). En ce qui concerne la partie (B'), cette dernière est également identique à la partie (B), à la seule différence de l'implant (2) dont la face de dessus (2b') est non plus convexe mais concave, en présentant un profil en creux complémentaire au profil en relief de la face de dessus (2b) de l'implant (2), relatif à la partie (B). On renvoie aux figures 14, 15, 16, 17 et 19, où l'on voit les parties (A) et (B) assujetties au corps vertébral (C3a) et les parties (A') et (B') assujetties au corps vertébral (C2a). Le profil convexe (2b) coopère avec le profil concave (2b').

Les avantages ressortent bien de la description, en particulier on souligne et on rappelle:
- le non blocage de l'articulation vertébrale considéré, après mise en place de la prothèse,
- la simplicité de réalisation,
- la facilité de mise en place,
- la possibilité de fixer la prothèse sur le corps vertébral de la vertèbre inférieure et/ou supérieure, en fonction de l'état de la partie cartilagineuse.

## Revendications

1. Prothèse rachidienne cervicale à glissement caractérisée en ce qu'elle comprend une partie (A) constituée d'un élément support (1) présentant une plaque cintrée (1b) pour correspondre très sensiblement au profil externe de l'un des corps vertébraux (C3a), ladite plaque (1b) délimitant deux pattes de fixation (1b1) (1b2), ledit élément (1) présente, dans un plan très sensiblement horizontal, une partie (B) sous forme d'un implant profilé (2), apte à coopérer aussi bien avec la surface articulaire fibro-cartilagineuse (C2b) du corps vertébral disposé en regard (C2a), qu'avec une partie complémentaire (B') que présente une autre partie (A') rendue solidaire dudit corps vertébral (C2a).

2. Prothèse selon la revendication 1, caractérisée en ce que la partie de l'élément support (1) présentant l'implant (2), est constituée par un anneau ouvert (1a) prolongé, à l'opposé de son ouverture (1a1) et dans un plan sensiblement orthogonal, par la plaque cintrée (1b).

3. Prothèse selon la revendication 1, caractérisée en ce que l'implant (2) présente un plot de centrage et de fixation (2a) apte à être emmanché à force dans l'épaisseur de l'anneau (1a).

4. Prothèse selon la revendication 1, caractérisée en ce que l'implant (2) a une forme générale, vue en plan, très sensiblement circulaire.

5. Prothèse selon la revendication 4, caractérisée en ce que la face de dessus de l'implant (2) a un profil en relief (2b) très sensiblement complémentaire au profil en creux (C2b) du corps vertébral (C2a) avec lequel il coopère.

6. Prothèse selon l'une quelconque des revendications 1 et 4, caractérisée en ce que la face de dessus de l'implant (2) relatif à la partie (B') a un profil en creux (2b') très sensiblement complémentaire au profil en relief (2b) de l'implant (2) relatif à la partie (B).

## Patentansprüche

1. Gleithalswirbelzwischenprothese, dadurch gekennzeichnet, daß sie einen Bereich (A) aufweist, der aus einem Halterungselement (1) besteht, das eine gewölbte Platte (1b) aufweist, die im wesentlichen dem externen Profil des Wirbelkörpers (C3a) entspricht, wobei die Platte (1b) zwei Befestigungsklauen (1b1) (1b2) abgrenzt, wobei das Element (1) einen Bereich (B) in einer im wesentlichen horizontalen Ebene aufweist, welcher als Untersatz für ein Implantationsprofil (2) gebildet ist und ebenso zum Zusammenwirken mit der gelenkigen Fibro-Cartilagina-Oberfläche (C2b) des Wirbelkörpers geeignet ist, welche im Schacht (C2a) angeordnet ist und welche einen komplementären Bereich (B`) sowie einen weiteren Bereich (A`) umfaßt, der am Wirbelkörper (C2a) befestigt ist.

2. Prothese nach Anspruch 1, dadurch gekennzeichnet, daß der Bereich des Halterungselements (1), welcher das Implantat (2) aufweist, durch einen offenen, länglichen Ring (1a), nämlich gegenüberliegend seiner Öffnung (1a1) und in einer im wesentlichen orthogonalen Ebene, durch die gewölbte Platte (1b)gebildet ist.

3. Prothese nach Anspruch 1, dadurch gekennzeichnet, daß das Implantat (2) einen Zentrierungs- und Fixierungsstift (2a) aufweist, der in die Dicke des Ringes (1a) zwangsweise einsteckbar ist.

4. Prothese nach Anspruch 1, dadurch gekennzeichnet, daß das Implantat (2) eine in der Ebene gesehene allgemein kreisförmige Form aufweist.

5. Prothese nach Anspruch 4, dadurch gekennzeichnet, daß die Fläche oberhalb des Implantats (2) ein erhabenes Profil (2b) aufweist, welches im wesentlichen komplementär zu einem vertieften Profil (C2b) des Wirbelkörpers (C2a) ist, mit dem es kooperiert.

6. Prothese nach einem der Ansprüche 1 und 4, dadurch gekennzeichnet, daß die Oberfläche des Implantats (2) relativ zum Abschnitt (B`) ein vertieftes Profil (2b`) aufweist, das im wesentlichen komplementär zu dem erhabenen Profil (2b) des Implantats (2) relativ zu dem Bereich (B) ist.

## Claims

1. A sliding cervical spinal prosthesis characterized in that it comprises a part (A) formed by a support element (1) having a plate (1b) curved to correspond very approximately with the external profile of one of the vertebrae (C3a), the said plate (1b) forming two fixing arms (1b1, 1b2), the said element (1) comprising a part (B) in a very approximately horizontal plane in the form of a profiled implant (2) adapted to cooperate equally well with the fibro-cartileginous joint surface (C2b) of the facing vertebra (C2a) as with a complementary part (B') which another part (A') fixed integrally to the said vertebra (C2a) provides.

2. A prosthesis according to claim 1, characterized in that the part of the support element (1) having the implant (2) is formed by an open ring (1a), extended opposite its opening (1a1) and in a substantially orthogonal plane by the curved plate (1b).

3. A prosthesis according to claim 1, characterized in that the implant (2) has a centring and fixing stud (2a) adapted to be force fitted in the thickness of the ring (1a).

4. A prosthesis according to claim 1, characterized in that the implant (2) has a very approximately circular general shape as viewed in plan,

5. A prosthesis according to claim 4, characterized in that the top face of the implant (2) has a raised profile (2b) very approximately complementary to the hollow profile (C2b) of the vertebra (C2a) with which it cooperates.

6. A prosthesis according to any one of claims 1 to 4, characterized in that the top face of the implant (2) pertaining to the part (B') a hollow profile (2b') very approximately complementary to the raised profile (2b) of the implant (2) pertaining to the part (B).
